# EUROPÄISCHE PATENTANMELDUNG

(11) **EP 1 398 012 A1**
(43) Veröffentlichungstag der Anmeldung: **17.03.2004**
(21) Anmeldenummer: 02019432.0
(22) Anmeldetag: 30.08.2002
(51) Int. Cl.: A61F 9/08

(54) **Verfahren und Vorrichtung zur Erzeugung intersensorischer Perceptions-Assoziationen**

(71) Anmelder: Rheinische Friedrich-Wilhelms-Universität Bonn, 53113 Bonn (DE)
(72) Erfinder: Rheinische Friedrich-Wilhelms-Universität Bonn, 53113 Bonn (DE)
(74) Vertreter: Hilleringmann, Jochen, Dipl.-Ing.

(57) **Zusammenfassung**

Die aus Vorrichtung und Verfahren bestehende Erfindung betrifft einen lernfähigen, Benutzer-spezifischen Signalwandler und Sinnesreizpaar-Speicher für die Wandlung von Sinnesreizen (SIV) für ein Sinnesorgan, z.B. S₁ in jeweils subjektiv als ähnlich empfundene, oder assoziierte Sinnesreize (SOV) für ein anderes Sinnesorgan, z.B. S₂ zur Auslösung von psychophysischen Perzepts oder Assoziationen P₂, mit einem Vector Translator Module (TM) und einem gekoppelten Learning Module (LM), wobei zunächst der Benutzer subjektiv in einer Lernprozedur ein durch SIV ausgelöstes Perzept P₁ mit dem durch SOV ausgelösten P₂ vergleicht und dem LM das psychophysische Vergleichsergebnis für eine iterative Optimierung des in TM erzeugten SOV signalisiert und wobei später das mit vielen als ähnlich bewerteten Sinnesreiz-Datenpaaren geladene TM in Kontakt mit dem Benutzer, oder autonom betrieben werden kann.

## Beschreibung

Die Erfindung betrifft ein Verfahren und eine Vorrichtung zur Erzeugung intersensorischer Perceptions-Assoziationen. Ferner betrifft die Erfindung auch ein Verfahren für den Betrieb eines Intersensory Perception Associator (IPA).

### Stand der Technik

1. Es sind Systeme zur Transformation von Sinnesreizen für ein Sinnesorgan auf Sinnesreize für ein anderes Sinnesorgan bekannt :
   US 2002/0067271 (22AUG00)
   US 5,097,326 (27JUL89)
   US 5,636,038 (24JUN96)
2. Es sind Systeme bekannt, dessen Funktion wahrnehmungsbasiert modifiziert werden:
   US 6,093,953 (04AUG98)
   US 6,400,989 (21FEB97)
   US 6,425,764 (12DEZ97)
   US 6,408,293 (09JUN99)
3. Es sind lernfähige Systeme bekannt:
   - Computational Intelligence - Imitating Life,
   - Eds. Zurada - Marks - Robinson
   - IEEE Press, New York, 1994
   - An Introduction to Neural and Electronic Networks
      Eds. Zornetzer - Davis - Lau
      Academic Press, New York, 1990
4. Es sind Systeme zur diskreten, in der Nähe befindlichen Personen nicht auffälligen, Signalübertragung bekannt:
   - Vibrator Signale von einem Mobil-Telefon oder Pager
   - Mini-Lautsprecher integriert im Hörorgan
   - In Brille eingespeist projektierter Bildschirm (Sony, Olympus)

### Nachteile des Standes der Technik

1. Das Spektrum diskret übertragbarer Nachrichten ist äußerst begrenzt.
2. Es können für die meisten Sinnesorgane keine psychophysisch korrespondierende Sinnesreize für andere Sinnesorgane gefunden werden z.B. Geruch als Surrogat für Klang, Taktiles Ereignis als Surrogat für Bild.
3. Sinnesreizmuster und dessen psychophysisch korrespondierende Reizmuster können nicht gespeichert, zugeordnet und abgerufen werden.
4. Der psychophysische Wahrnehmungsraum kann bisher nicht annähernd adäquat ausgelotet werden, was für die Entwicklung diverser Geräte mit Mensch-Maschine Schnittstelle () und für die Entwicklung von Medizin - Technik - Produkte benötigt wird.
5. Taktile Nachrichtengeber wie z.B. Pager, oder Vibrationssignalgeber an Mobiltelefonen sind beschränkt auf reine Hinweissignale ohne die Fähigkeit zur Übermittlung von komplexeren Nachrichten.

### Erfindung

Diese Nachteile sollen durch die hier offenbarte Erfindung beseitigt oder zumindest erheblich reduziert werden, und zwar wie in den Ansprüchen 1, 4 und 10 angegeben. Die Unteransprüche betreffen bevorzugte Weiterbildungen der Erfindung.

### Definitionen

**Perzept:** Ein Perzept ist das, was wahrgenommen wird. Es ist weder der physikalische Gegenstand (distaler Reiz) noch sein Abbild in einem Rezeptor (proximaler Reiz). Vielmehr handelt es sich um das erfahrene (phänomenale) Ergebnis des gesamten Wahrnehmungsprozesses.

**Spatio-temporal:** Unter spatio-temporalen Funktionen werden Funktionen verstanden, die von Zeit und Ort abhängen.

**Physical Pattern Space:** Unter einem Physical Pattern Space wird im folgenden der Raum aller physikalischen Sinnesreizmuster verstanden.

**Physikalisches Sinnesreizmuster:** Unter einem physikalischen Sinnesreizmuster wird im folgenden ein für ein oder mehrere Sinnesorgane adäquates Reizmuster verstanden, das nach Umwandlung (in den entsprechenden Rezeptoren) eine Sinneswahrnehmung bzw. ein Perzept eines Objektes oder eines Begriffes auslöst.

**SIV (Sensory Input Vector):** Unter einem SIV wird im folgenden ein physikalisches Sinnesreizmuster verstanden.

**SOV (Sensory Output Vector):** Unter einem SOV wird ein physikalisches Sinnesreizmuster verstanden, der von TM generiert über die Signalgeber ST ausgegeben wird.

**SR (Signal Receiver):** Unter einem SR wird ein Sinnesorgan-spezifischer Aufnehmer verstanden, der ein Teil eines physikalischen Sinnesreizmusters z.B. elektronisch umwandelt, so dass der überwiegende Teil der Informationen des physikalischen Sinnesreizmusters für eine spätere Reproduktion des Sinnesreizmusters für das spezifische Sinnesorgan erhalten bleibt, beispielsweise ein Mikrophon, eine Video-Kamera, eine elektrische Nase, etc. Ein SR kann auch weiterverarbeitende Elemente wie z.B. Filter, Verstärker, Analog-Digitalwandler, etc beinhalten.

**ST (Signal Transmitter):** Unter einem ST wird ein Sinnesorgan-spezifischer Wandler verstanden, der z.B. elektrische Signale in physikalische Sinnesreizmuster umwandelt, wobei ein möglichst großer Raum des mit dem jeweiligen Sinnesorgan aufnehmbaren Raumes ausgefüllt werden kann. Ein ST kann auch vorverarbeitende Elemente beinhalten, z.B. Digital-Analog-Wandler, Verstärker, Filter, etc. Beispiele für STs sind Lautsprecher, Bildschirme, taktile Aktuatoren, etc.

**Psychophysical Perception Space:** Unter dem Psychophysical Perception Space wird der Wahrnehmungsraum verstanden.

### Ziele der Erfindung

1. Personen-spezifische Wandlung von Sinnesreizmustern mit Wechsel des empfangenden Sinnesorgans.
2. Personen-spezifische Detektion von Sinnesreizmuster-Paaren, die überwiegend oder exklusiv über verschiedene Sinnesorgane angeboten werden, jedoch hinreichend ähnliche, psychophysische Wahrnehmungen oder Assoziationen auslösen.
3. Personen-spezifisches Lernen von Wahrnehmungsassoziationen für biomedizinische Anwendungen.
4. Personen-spezifisches Lernen von Wahrnehmungsassoziationen in Wirtschaft und Technik.
5. Diskrete, von in der Nähe befindlichen Menschen nicht bemerkbare Kommunikation über körpernahe Signalübertrager in Wirtschaft und Technik.
6. Personen-spezifische Wiedergabe multimodaler Sinnesreizmuster.
7. Personen-spezifische, automatische Exploration des Wahrnehmungsraumes und des Assoziationsraumes.

### Vorteile der Erfindung

Gegenüber herkömmlichen Systemen zur Übertragung von Sinneskanal-spezifischen Informationen auf ein anderes Sinnesorgan hät das offenbarte System (IPA) den Vorteil, dass die Ausgangssignale (SOV) durch die Einstellung über einen wahrnehmungsbasierten Lernprozess optimal an die Wahrnehmung eines Benutzers angepasst werden.

Nach dem Lernprozess können die Ausgangssignale (SOV) direkt vom Benutzer korrekt assoziiert und interpretiert werden, denn sie sind nach seinen Vorstellungen entsprechend komponiert worden. Dies hat den Vorteil, dass der Benutzer die durch die Signale zu übertragenden Informationen sehr schnell aufnehmen kann, und dass es dem Benutzer möglich ist, eine große Anzahl von SOVs über ein anderes Sinnesorgan zu "verstehen".

Außerdem wird die Aufnahmefähigkeit der Sinnesorgane optimal ausgenutzt, und es werden neue Sinnesreize generiert, die bislang nicht vorstellbar waren und deshalb bei herkömmlichen Verfahren nicht zur Anwendung kamen.

Weiterhin ist ein Vorteil gegenüber bekannten Verfahren, dass nicht nur relative Abstände von Wahrnehmungen eines Sinneskanals übersetzt werden können in relative Abstände von Wahrnehmungen eines anderen Sinneskanals, sondern dass eine Art Abbildungsfunktion zwischen Perzepten verschiedener Sinneskanäle gefunden werden kann. Auf diese Weise wird es möglich, über eine mit einem lernendem System gefundene Abbildungsvorschrift Informationen intersensorisch zu übertragen.

### Vorteilhafte Ausführungen

Eine vorteilhafte Ausführung des IPA besteht darin, dass in einem personenspezifischen, wahrnehmungsbasierten Lernvorgang SOV für Sinneskanal S₂ gefunden wird, dessen Perzept P₂ der Benutzer mit Perzept P₁ assoziiert, das er bislang mit einem anderen Sinnensorgan empfangen hat.

Eine vorteilhafte Ausführung des IPA besteht darin, dass in einem personenspezifischen, wahrnehmungsbasierten Lernvorgang SOV für Sinneskanal S₂ gefunden wird, dessen Perzept P₂ der Benutzer auch mit Perzept P' assoziiert, das er in seiner Erinnerung gespeichert hat.

Eine vorteilhafte Ausführung des SIV besteht darin, dass die Auswahl des SIV durch das Learning Module (LM) oder durch den Benutzer des IPAs getroffen werden kann.

Vorteilhafte Ausgestaltungen eines Sinnesorgan-spezifischen Sinnesreizaufnehmers SR bestehen darin, dass der SR aus einem Sensor besteht, der z.B. auditorische (Mikrofon), visuelle(Videokamera), olfaktorische, oder haptische Ereignisse registrieren kann und dass eine Weiterverarbeitung der Signale z.B. Vorverstärkung oder Digitalisierung vorgesehen ist.

Vorteilhafte Ausgestaltungen des Speicher Moduls (SM) in TM bestehen darin, dass SM aus einem in der elektronischen Datenverarbeitung üblichen Speicher, z.B. Digitalchip oder magnetische Datenspeicher besteht.

Vorteilhafte Ausgestaltungen eines Sinnesorgan-spezifischen Sinnesreiz-Signalgebers ST bestehen darin, dass der ST z.B. aus einem Lautsprecher, einem LCD Display, einem Array von Molekülspendern zur Komposition von Geruchsmustern, oder einem taktilen Aktuator-Array besteht.

Weiterhin kann in einer vorteilhaften Ausführung in TM das Mapping Module (MM) z.B. in Form von einer Lookup-Table realisiert werden, die die im Lernverfahren gefundenen Parametersätze des FMs, deren SOV eine Wahrnehmung P₂ mit ausreichender Ähnlichkeit zu P₁ hervorruft, mit den durch die SRs aufgenommenen Signale von SIV verknüpft.

In einer weiteren vorteilhaften Ausführung kann MM zusätzlich eine vorverarbeitende Struktur beinhalten, die z.B. eine Klassifizierung der von SR kommenden Eingangssignale, oder eine Filterung vornimmt.

Auch kann in einer vorteilhaften Ausgestaltung des MM dieses die Identität für Teile des Eingaberaums oder für den gesamten Eingaberaum darstellen, so dass die von SR gelieferten Signale direkt an FM weitergegeben werden.

Eine vorteilhafte Ausführung des Funktionsgenerator Moduls (FM) besteht darin, dass FM aus einer Kombination von unterschiedlichen Signalgenerator-Komponenten, wie z.B. rückgekoppelte neuronale Netze, programmierbare digitale Funktionsgeneratoren, oder rückgekoppelte Filter, besteht, die über einen Satz von Parametern zur Erzeugung diverser Zeitfunktionen als Funktionsgenerator Vektoren (FGV) eingestellt werden können.

Ferner besteht eine vorteilhafte Ausführung darin, dass die von FM erzeugten Funktionsgenerator Vektoren (FGV) den Signalgebern ST in Form von Zeit-Funktionen, zugeführt werden und dort in Sinnesorgan-spezifische Signal Output Vektoren (SOV) umgewandelt werden.

In einer vorteilhaften Ausführung hat FM zur Speicherung von Daten (Parametersätzen oder FGVs) Zugriff auf das Speicher Modul (SM).

Weiterhin besteht eine vorteilhafte Ausführung darin, dass FM von MM Parametersätze erhält, oder dass FM von MM eine Adresse eines Parametersatzes in SM erhält und dass FM dem Mapping Module MM bei Bedarf Angaben zu dem aktuellen Parametersatz macht.

Eine vorteilhafte Ausführung der Lernprozedur (LP) besteht darin, dass LP auf evolutionären, genetischen Algorithmen, oder Reinforcement Learning basiert.

Weiterhin kann eine vorteilhafte Ausführung für LP darin bestehen, dass mit Hilfe des Feedbacks des Benutzers iterativ das SOV optimiert wird, mit dem Ziel, dass das durch SOV erzeugte Percept P₂ als hinreichend ähnlich assoziiert wird zu dem Percept P₁.

Auch kann in einer weiteren vorteilhaften Ausführung das Learning Module LM über TMC direkt Einfluss auf den Parametersatz haben, in einer anderen Ausführung kann das Learning Module (LM) nur Richtungsvorschläge zur Veränderung von Parametersätzen an das MM oder FM über TMC geben.

Eine vorteilhafte Ausgestaltung der Wahl des Sinnesorgans für S₂ besteht darin, dass der Benutzer das Sinnesorgan S₂ explizit vor der Lernphase benennt.

Eine weiter vorteilhafte Ausgestaltung kann darin bestehen, dass der Benutzer das Sinnesorgan S₂ während der Lernphase benennt.

Eine weiter vorteilhafte Ausgestaltung kann darin bestehen, dass das Sinnesorgan S₂ während der Lernphase vom LM bestimmt wird.

Eine vorteilhafte Ausgestaltung des Motor Output Vector (MOV) besteht darin, dass das zugeordnete Vergleichsergebnis durch ein motorisches Feedback des Benutzers eingegeben wird, z.B. durch eine Handbewegung ein Schaltpult bedient wird, oder eine verbale Rückmeldung gegeben wird.

Weiterhin kann eine vorteilhafte Ausgestaltung für MOV darin bestehen, dass das Vergleichsergebnis aus einer physiologisch abgeleiteten Größe gewonnen wird, wie z.B. aus dem spezifischen Hautwiderstand einer Körperpartie des Benutzers.

Eine vorteilhafte Ausgestaltung des psychophysischen Vergleichs von P₁ mit P₂ besteht darin, dass der Benutzer aus einer vorgegebenen Menge von Vergleichsmustern die n-Besten auswählen muss.

Weiterhin kann eine vorteilhafte Ausgestaltung des Vergleichs darin bestehen, dass der Benutzer eine subjektive Beurteilung der angebotenen Vergleichsmuster auf einer vorgegeben Skala (z.B. Schlecht, Mittel, Gut, Sehr Gut) durchführen muss.

Eine vorteilhafte Ausgestaltung des Learning Module Interface (LMI) besteht darin, dass der MOV durch ein Tastenfeld, ein Mikrophon, eine Videokamera oder einen Sensor, der eine physiologischen Größe erfassen kann, aufgenommen wird, eine Weiterverarbeitung durchgeführt wird und als Vektor an das LM weitergeleitet wird.

Eine vorteilhafte Ausgestaltung der Speicherung der benötigten Daten zur Replikation von SIV besteht darin, dass die Daten am Ausgang der SRᵢ im SM in analoger oder digitaler Form gespeichert werden.

Eine weitere vorteilhafte Ausgestaltung der Speicherung der benötigten Daten zur Replikation von SIV besteht darin, dass die Klassifizierungsmerkmale abgespeichert werden.

Eine vorteilhafte Ausgestaltung der Speicherung der benötigten Daten zur Replikation von SOV besteht darin, dass sowohl die Parameter des FM, als auch die charakterisierenden Eigenschaften des FM, die zur Erzeugung des SOV benötigt werden, im SM gespeichert werden.

Eine weitere vorteilhafte Ausgestaltung kann darin bestehen, dass die FGV, die zur Erzeugung von SOV an STᵢ übermittelt wurden, im SM gespeichert werden.

Eine vorteilhafte Ausgestaltung der Zuordnung von SIV mit SOV besteht darin, dass für diese Zuordnung eine Lookup - Table benutzt wird, wobei eine von SIV abgeleitete digitale Größe als eine Adresse interpretiert wird und die zur Erzeugung von SOV benötigten Parameter an dieser Adresse abgelegt sind.

Eine vorteilhafte Ausgestaltung der Steuerung der Speicherung und des Wiederaufrufes von SIV und SOV besteht darin, dass MM, FM und LM, letzteres über TMC, durch Steuersignale dem SM signalisieren, ob aus dem Speicher gelesen bzw. geschrieben werden soll und daraufhin die entsprechenden Daten über Signalleitungen erhalten bzw. zur Verfügung stellen.

Eine vorteilhafte Ausgestaltung der Wandlung von SIV zu SOV im Normalmodus besteht darin, dass unter Verwendung einer Lookup-Table im MM eine Adresse vom SIV abgeleitet wird und der entsprechende Eintrag der Loopup-Table an das FM übermittelt wird, so dass ein SOV erzeugt wird.

Eine weitere vorteilhafte Ausführung der Wandlung von SIV zu SOV im Normalmodus besteht darin, dass falls bei Präsentation von einem SIV nur eine Ähnlichkeit zu einem bekannten SIV gegeben ist, das SOV des bekannten SIV ausgegeben wird.

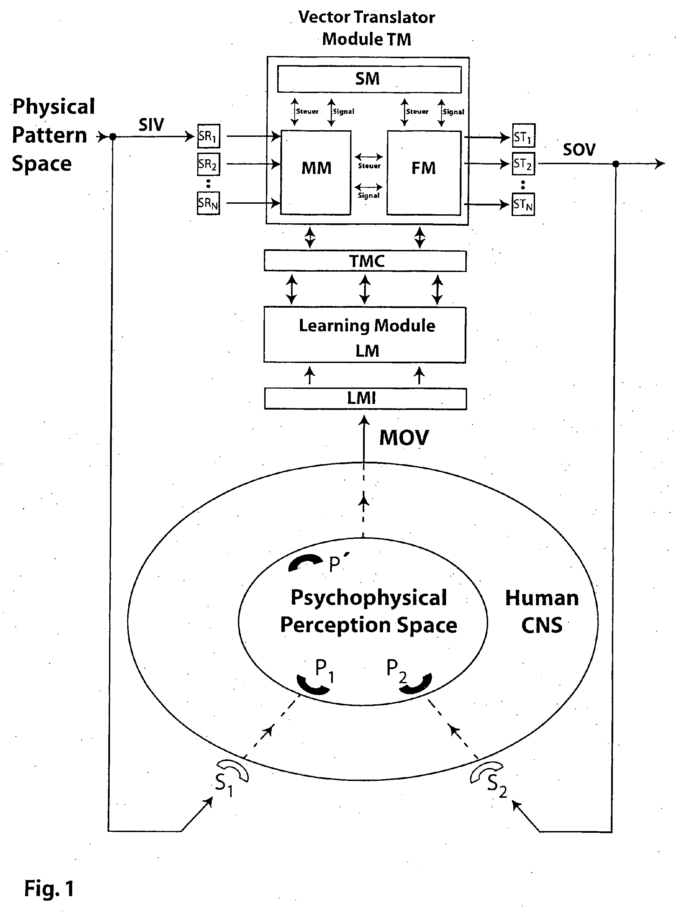

### Beschreibung der Fig. 1

IPA umfasst erstens ein Vector Translator Module (TM) zur Umwandlung von Sinnesreizen (SIV) für ein Sinnesorgan S₁ in psychophysikalisch korrespondierende Sinnesreize (SOV) für ein anderes Sinnesorgan S₂, und zweitens ein Learning Module (LM) mit einer Lernprozedur (LP).

TM und LM arbeiten in Verbindung mit einer Benutzerperson, deren Gehirnfunktion (CNS = Zentralnervensystem) hier durch zwei ineinander geschachtelte Ellipsen angedeutet ist. Dabei befinden sich die Sinnesreizaufnehmer der Sinneseingänge S₁, S₂ unten am Außenrand.

Hirnfunktions-Ausgangssignale bzw. physiologische Körperfunktionssignale können aus der oberen Ellipsenhälfte entnommen/gemessen werden: hier angedeutet als Motor Output Vector (MOV). Der aus prinzipiellen Gründen nicht zugängliche Wahrnehmungsraum (Psychophysical Pattern Space) ist innerhalb der inneren Ellipse angedeutet.

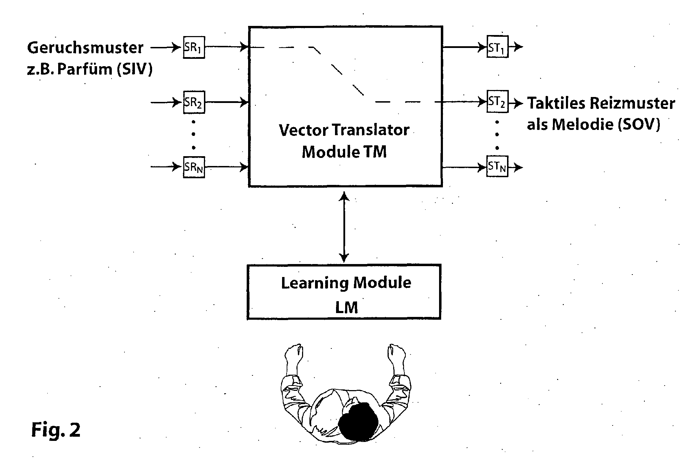

Der Benutzer empfängt SIV und SOV über zwei verschiedene Sinnesorgane S₁ und S₂ und hat entsprechend je eine zugehörige Wahrnehmung oder Assoziation, die hier Perzept P₁ (z.B. olfaktorischer Sinneseindruck, Geruch eines Parfüms) und Perzept P₂ (taktiler Sinneseindruck als Melodie) genannt ist (siehe Fig. 2). Aufgabe von Vorrichtung und Verfahren (IPA) sind u.a.:
1. Iterative Lernen-basierte Suche nach Sinnesreiz-Paaren für zwei Sinnesorgane, deren zugehörige Perzepte P₁, P₂ als hinreichend ähnlich wahrgenommen bzw. assoziiert werden.
2. Übersetzung von Sinnesreizen von einem Sinnesorgan zum anderen.

### Beschreibung der Fig. 2

In Fig.2 ist eine Beispielanwendung für den IPA dargestellt. Als Sinnesreiz-Eingangsvektor (SIV) ist ein Geruchsmuster gezeigt, das vom Riechorgan des Benutzers aufgenommen wird und gleichzeitig mittels eines Geruchssensor-Arrays (SR₁) in ein digitales Signal gewandelt und im TM gespeichert wird. TM generiert nun seinerseits einen Sinnesreiz-Ausgangsvektor (SOV) über ein taktiles Aktuator-Array (ST₂). SOV bewirkt nun eine taktile Wahrnehmung und Assoziation bei dem Benutzer. Diese psychophysische Wahrnehmung oder Assoziation hat der Benutzer mit der durch das Geruchsmuster hervorgerufenen Assoziation während einer Lernprozedur (LP) mit LM als hinreichend ähnlich ausgewählt.

## Patentansprüche

1. Intersensory Perception Associator (IPA), **gekennzeichnet dadurch, dass** a) eine Personen-spezifische Wandlung eines Sinnesreiz Eingangsvektors (SIV) für mindestens überwiegend ein Sinnesorgan (S₁) in einer Lernprozedur (LP) in einen Wahrnehmungs-basiert korrespondierenden Sinnesreiz Vektor (SOV) für zumindest überwiegend ein anderes Sinnesorgan (S₂) erfolgt, dass b) ein lernfähiges Vector Translator Module (TM) bestehend aus mindestens einem Sinnesorgan-spezifischen Sinnesreizaufnehmer (SR) am Eingang von TM, einem Speicher Modul (SM), einem Mapping Module (MM) und einem Funktionsgenerator Modul (FM) und mindestens einem Sinnesorgan-spezifischen Sinnesreiz-Signalgeber (ST) am Ausgang von TM vorgesehen ist, wobei TM eine Schnittstelle (TMC) zur TM-Funktionssteuerung zugeordnet ist und FM Sinnesorgan-spezifische Signale (FGV) für die ST erzeugt, dass c) ein Learning Module (LM) vorgesehen ist, wobei erstens eine Signalverbindung vom LM-Ausgang zu TMC zugeordnet ist und wobei zweitens eine Eingabe-Schnittstelle (LMI) zur Benutzer-spezifischen Dateneingabe zugeordnet ist, dass d) TM die Abbildung einer Pluralität von Sinnesreiz-Eingangsvektoren (SIV) auf individuell korrespondierende Sinnesreiz-Ausgangsvektoren (SOV) festlegt, wobei erstens die Abbildung (M) von SIV in SOV nicht vorgegeben ist und wobei zweitens M nicht beschrieben ist und wobei drittens SOV überwiegend oder exklusiv ein anders Sinnesorgan S₂ stimuliert, als das überwiegend oder exklusiv von SIV stimulierte S₁, dass e) eine Lernprozedur (LP) vorgesehen ist zur Benutzer-spezifisch festzulegenden Zuordnung von SIV zu SOV, wobei erstens ein Benutzer die durch SIV hervorgerufene Wahrnehmung oder Assoziation als psychophysisches Perzept (P₁) vergleicht mit einer durch SOV hervorgerufenen Wahrnehmung oder Assoziation als psychophysisches Perzept (P₂), wobei zweitens der Benutzer seinen subjektiven, psychophysischen Bewertungsvergleich von (P₁) mit (P₂) über einen von ihm ausgelösten Outputvektor (MOV) an LM übermittelt und wobei drittens SOV iterativ solange im Dialog mit dem Benutzer verstellt wird, bis der Benutzer hinreichende Korrespondenz zwischen (P₁) mit (P₂) signalisiert.

2. Intersensory Perception Associator nach Anspruch 1 **gekennzeichnet dadurch, dass** erstens am Mapping Module (MM) separate Eingänge den Ausgängen der dem TM zugeordneten Sinnesreizaufnehmer (SRᵢ) zugeordnet sind, dass zweitens zwischen MM und dem Funktionsgenerator Modul (FM) eine Signalkopplung und eine Steuerungskopplung vorgesehen ist und dass drittens zwischen MM und dem Learning Module (LM) via die Schnittstelle TMC eine Signalkopplung und eine Steuerungskopplung vorgesehen ist.

3. Intersensory Perception Associator nach einem der vorherigen Ansprüche **gekennzeichnet dadurch, dass** in TM ein Funktionsgenerator Modul (FM) zur Erzeugung von Funktionsgeneratorvektoren (FGV) vorgesehen ist, wobei erstens FM eine Schnittstelle zur Signalkopplung und zur Steuerungskopplung mit MM vorgesehen ist, wobei zweitens FM ein Signalweg zum TMC zugeordnet ist und wobei drittens FM Signalwege zu den Signalgebern STⱼ in TM zugeordnet sind.

4. Verfahren zum Betrieb eines Intersensory Perception Associator's nach einem der vorherigen Ansprüche **gekennzeichnet dadurch, dass** im Betriebszustand: F-Learning erstens in LM eine Lernprozedur (LP) eingesetzt wird, wobei LP insbesondere einen Algorithmus für ,Nichtüberwachtes Lernen' darstellt, dass LP zweitens die Funktion von FM beeinflusst, dass LP drittens eine iterative Veränderung eines in FM erzeugten FVG bewirkt, wobei FGV über ein zugehörig ausgewähltes ST₂ in ein SOV gewandelt und dem Benutzer während des Lernvorganges präsentiert wird.

5. Verfahren zum Betrieb eines Intersensory Perception Associator's nach einem der vorherigen Ansprüche **gekennzeichnet dadurch, dass** die Wahl des Sinnesorgans S₂, für welches P₂ optimiert werden soll, von einem Benutzer getroffen wird.

6. Verfahren zum Betrieb eines Intersensory Perception Associator's nach einem der vorherigen Ansprüche **gekennzeichnet dadurch, dass** im Betriebszustand: F-Learning erstens der Benutzer einen SIV als Perzept (P₁) von S₁ wahrnimmt, dass zweitens der Benutzer einen SOV als Perzept (P₂) von S₂ wahrnimmt und dass drittens aus dem psychophysischen Vergleich des Benutzers von (P₁) mit (P₂) das Vergleichergebnis durch Benutzer-spezifische Hirnfunktionen in einen dem Vergleichsergebnis zugeordneten Outputvektor (MOV) gewandelt wird und dass viertens durch MOV als Eingabewert für LM die Lernprozedur (LP) gesteuert wird.

7. Verfahren zum Betrieb eines Intersensory Perception Associator's nach einem der vorherigen Ansprüche **gekennzeichnet dadurch, dass** im Betriebszustand: F-Learning im Speicher Modul (SM) erstens zur späteren Replikation von SOV benötigte Daten gespeichert werden und dass zweitens in SM zur späteren Replikation von SIV benötigte Daten gespeichert werden.

8. Verfahren zum Betrieb eines Intersensory Perception Associator's nach einem der vorherigen Ansprüche **gekennzeichnet dadurch, dass** im Betriebszustand: F-Learning die Module MM und LM die Zuordnung der jeweiligen Datenpaare: SIV mit SOV bzw. der SR₁ Output zu SIV mit FGV als ST₂ Input für SOV einschließlich der Speicherung und des Wiederaufrufes steuern.

9. Verfahren zum Betrieb eines Intersensory Perception Associator's nach einem der vorherigen Ansprüche **gekennzeichnet dadurch, dass** im Betriebszustand: F-Operation TM erstens eine Wandlung von SIV an Sinnesorgan S₁ in ein SOV an ein zu einer von S₁ unterschiedlichen Sinnesmodalität gehöriges Sinnesorgan S₂ durchführt und dass zweitens die durch SIV hervorgerufene Wahrnehmung oder Assoziation P₁ subjektiv bei demselben Benutzer, der während F-Learning TM für seine Wahrnehmung optimal eingestellt hat, nun während F-Operation ohne notwendige Mitbenutzung von LM eine Präsentation von SOV eine zu P₁ hinreichend ähnliche Wahrnehmung oder Assoziation P₂ hervorruft.

10. Verfahren zur Erzeugung intersensorischer Perceptions-Assoziationen, bei dem a) eine Personen-spezifische Wandlung eines Sinnesreiz Eingangsvektors (SIV) für mindestens überwiegend ein Sinnesorgan (S₁) in einer Lernprozedur (LP) in einen Wahrnehmungs-basiert korrespondierenden Sinnesreiz Vektor (SOV) für zumindest überwiegend ein anderes Sinnesorgan (S₂) erfolgt, dass b) ein lernfähiges Vector Translator Module (TM) bestehend aus mindestens einem Sinnesorgan-spezifischen Sinnesreizaufnehmer (SR) am Eingang von TM, einem Speicher Modul (SM), einem Mapping Module (MM) und einem Funktionsgenerator Modul (FM) und mindestens einem Sinnesorgan-spezifischen Sinnesreiz-Signalgeber (ST) am Ausgang von TM vorgesehen ist, wobei TM eine Schnittstelle (TMC) zur TM Funktionssteuerung zugeordnet ist und FM Sinnesorgan spezifische Signale (FGV) für die ST erzeugt, dass c) ein Learning Module (LM) vorgesehen ist, wobei erstens eine Signalverbindung vom LM-Ausgang zu TMC zugeordnet ist und wobei zweitens eine Eingabe-Schnittstelle (LMI) zur Benutzer-spezifischen Dateneingabe zugeordnet ist, dass d) TM die Abbildung einer Pluralität von Sinnesreiz-Eingangsvektoren (SIV) auf individuell korrespondierende Sinnesreiz-Ausgangsvektoren (SOV) festlegt, wobei erstens die Abbildung (M) von SIV in SOV nicht vorgegeben ist und wobei zweitens M nicht beschrieben ist und wobei drittens SOV überwiegend oder exklusiv ein anders Sinnesorgan S₂ stimuliert, als das überwiegend oder exklusiv von SIV stimulierte S₁, dass e) eine Lernprozedur (LP) vorgesehen ist zur Benutzer-spezifisch festzulegenden Zuordnung von SIV zu SOV, wobei erstens ein Benutzer die durch SIV hervorgerufene Wahrnehmung oder Assoziation als psychophysisches Perzept (P₁) vergleicht mit einer durch SOV hervorgerufenen Wahrnehmung oder Assoziation als psychophysisches Perzept (P₂), wobei zweitens der Benutzer seinen subjektiven, psychophysischen Bewertungsvergleich von (P₁) mit (P₂) über einen von ihm ausgelösten Outputvektor (MOV) an LM übermittelt und wobei drittens SOV iterativ solange im Dialog mit dem Benutzer verstellt wird, bis der Benutzer hinreichende Korrespondenz zwischen (P₁) mit (P₂) signalisiert.
